# EUROPEAN PATENT APPLICATION

(11) **EP 2 784 473 A1**
(43) Date of publication of application: **01.10.2014**
(21) Application number: 12851303.3
(22) Date of filing: 20.11.2012
(51) Int. Cl.: G01N 1/28, C12M 3/08

(54) **TISSUE SEGMENTATION APPARATUS, CELL SORTING APPARATUS, CELL SORTING SYSTEM, TISSUE DISPLAY SYSTEM, SUBSTRATE, EXTENDIBLE MEMBER, TISSUE SEGMENTATION METHOD, AND CELL SORTING METHOD**

(30) Priority: 25.11.2011 JP 2011257772
(71) Applicant: Olympus Corporation, Shibuya-ku Tokyo 151-0072 (JP)
(72) Inventor: MORIMOTO, Nobuhiko, Tokyo 151-0072 (JP)
(74) Representative: Schicker, Silvia
(86) International application number: PCT/JP2012/080106
(87) International publication number: WO 2013/077337

(57) **Abstract**

The present invention divides a section of a biological tissue into fragments in a size of including a sufficient amount of cells with a simple structure and a simple operation. The present invention provides a tissue dividing apparatus including: a substrate dividable into a plurality of small pieces (20a), and having a surface to which a section (A) of a biological tissue can be pasted; and a dividing unit (22, 23) that divides the substrate into a plurality of small pieces (20a), and thereby divide the section (A) pasted on the surface into fragments each having a substantially equivalent shape to a shape of each small piece (20a).

## Description

### {Technical Field}

The present invention relates to a tissue dividing apparatus, a cell collection apparatus, a cell collecting system, a tissue displaying system, a substrate, an expandable member, a tissue dividing method, and a cell collecting method.

### {Background Art}

Heretofore, the Laser Microdissection (LMD) method has been known as a technique to pick up a micro-region of about several tens microns by cutting it out from a tissue section for use in pathological diagnosis and the like (For example, see Non Patent Literature 1.). In the LMD method, the micro-region of the section to be picked up is irradiated with UV laser, by which the micro-region is cut out from the section.

### {Citation List}

### {Non Patent Literature}

### {NPL 1}

Leica MICROSYSTEMS, "Leica LMD 6500 Leica LMD 7000", P.2, [online], [searched on August 30, 2012], Internet < URL:http://www.leica-microsystems.com/fileadmin/downloads/Leica%20LMD7000/Brochures /LMD6500_7000_JP.pdf>

### {Summary of Invention}

### {Technical Problem}

However, in the case where a test, particularly a genetic test, is desired to be conducted with use of a minute biological sample, such as nucleic acid, a gene, amino acid, peptide, protein, lipid, and a sugar chain, extracted from cells contained in a minute fragment that has been prepared by cutting out a region where specific cells, such as cancer cells, exist from a section; the number of cells contained in the fragment having been picked up with the LMD method is too small to extract a sufficient amount of the gene necessary for the test. Accordingly, a large number of sections have to be subject to microdissection to collect a sufficient number of cells. This causes a problem in that large amounts of labor and time are required for conducting one genetic test.

On the other hand, it can be considered to increase the area of the fragment to be cut out, or to increase the thickness of the section to be cut out from the biological tissue. However, if the area of the fragment cut out by the LMD method is increased, it is necessary to increase the size of the laser-scannable region, or to increase the laser output so that the fragment having been cut out by laser scanning can be blown off by the pressure of laser. This leads to a disadvantage in that the scale of the device structure has to be much enlarged and also the price of the device has to be raised. In addition, if the thickness of the section is increased, the disadvantage is that it is necessary to increase the laser output after all so as to cut out the section by laser.

An object of the present invention, which has been made in order to solve the problems according to the conventional art, is to provide a tissue dividing apparatus, a cell collection apparatus, a cell collecting system, a tissue displaying system, a substrate, an expandable member, a tissue dividing method, and a cell collecting method that are capable of dividing a section of a biological tissue into fragments in a size of including a sufficient amount of cells, and picking up the fragments with a simple structure and a simple operation.

### {Solution to Problem}

In order to achieve the above-mentioned object, the present invention provides the following solutions.

The first aspect of the present invention is a tissue dividing apparatus including: a substrate having a surface to which a section of a biological tissue can be pasted, and dividable into a plurality of small pieces; and a dividing unit that divides the substrate into the plurality of small pieces, to thereby divide the section pasted on the surface into fragments each having a substantially equivalent shape to a shape of each small piece.

According to the first aspect of the present invention, through a simple configuration and a simple operation of dividing the substrate to which the section of the biological tissue is pasted into the plurality of small pieces with the dividing unit, the section pasted on the substrate together with this substrate can be divided into fragments each having substantially the same shape as that of each small piece, and the fragments pasted on the small pieces together with these small pieces can be picked up. The section is mechanically divided together with the substrate, so that the thickness of the section and the size of the fragment are not so strictly restricted as in the LMD method. Accordingly, increase in thickness of the section, or enlargement of the size of the small piece allows the section to be divided into the fragments having a size of including a sufficient amount of cells.

In the above first aspect, the substrate may be formed of an assembly of the plurality of small pieces that are divided in advance, and the dividing unit may divide the substrate by applying a force to the substrate to relatively move the small pieces with respect to one another. Alternatively, in the above first aspect, the substrate may be provided with grooves for segmenting the substrate into the plurality of small pieces, and the dividing unit may divide the substrate by applying a force to the substrate to relatively move the small pieces with respect to one another.

Through this configuration, the substrate can be easily divided into small pieces each having a predetermined shape by applying a force of relatively moving the small pieces with respect to one another to the substrate.

In the above first aspect, the dividing unit may apply a force to the substrate to relatively move the plurality of small pieces with respect to one another in a crossing direction to the surface. In this case, the dividing unit may move a part of the plurality of small pieces in the crossing direction.

Through this configuration, it is possible to move the part of the small pieces in the normal direction of the substrate surface such that the part of the small pieces is divided from the other small pieces, to thereby cut off the part of the section pasted on the surface of the part of the small pieces.

In this configuration, the part of the small pieces may include at least one small piece to which a region to be picked up of the section is pasted.

In this configuration, the dividing unit may include a support member for supporting at least one of a surface to which the section on the part of the small pieces is pasted, and an opposite surface to the surface, and the support member may be moved in the crossing direction, to thereby move the part of the small pieces in the crossing direction.

Through this configuration, it is possible to easily move only the part of the small pieces in the crossing direction to the surface.

In the configuration of including the aforementioned supporting member, the dividing unit may further include a dividing auxiliary unit that assists dividing of the section pasted on the part of the small pieces. In this case, the dividing auxiliary unit may include a substantially cylindrical member which holds the substrate between the supporting member and the cylindrical member with an end surface of the cylindrical member opposite to the substrate, and of which end surface is provided with a hole into which the part of the small pieces moved by the support member is insertable, and an edge between the end surface and an inner surface of the hole may assist the dividing of the section.

Through this configuration, in the process of inserting the small piece into the hole of the cylindrical member, the section pasted on this small piece can be more securely cut off.

In the configuration of including the aforementioned support member, the dividing unit may press the one surface of the small piece using the support member in the crossing direction.

Through this configuration, it is possible to more easily move the part of the small pieces.

In the configuration of pressing the one surface of the small piece with the aforementioned support member, the support member may support the surface to which the section is pasted via the section, and a protective member may be provided between the support member and the section so as to cover and protect the surface of the section.

This configuration prevents the support member from coming into direct contact with the section, to thereby prevent substances such as biological molecules included in one fragment from moving to (contaminating) another fragment via the support member even if a plurality of fragments are picked up successively using the identical support member.

The configuration of including the aforementioned support member may be equipped with an attracting member that is disposed between the support member and the opposite surface of the part of the small pieces and attracts the opposite surface, and the dividing unit may pull the opposite surface of the part of the small pieces in the crossing direction using the support member.

Through this configuration, it is possible to easily move the part of the small pieces.

In the configuration of including the aforementioned support member, the dividing unit may include two support members that are oppositely disposed to each other with the substrate held therebetween and thereby support the surface of the small pieces to which the section is pasted and the opposite surface, and the two support members may integrally move in the vertical direction.

The second aspect of the present invention is a cell collection apparatus including: a substrate having a surface to which a section of a biological tissue can be pasted, and dividable into a plurality of small pieces; a dividing unit that divides the substrate into the plurality of small pieces, and thereby divides the section pasted on the surface into fragments each having a substantially equivalent shape to a shape of each small piece; and a pickup unit that picks up the fragments of the section divided by the dividing unit.

The third aspect of the present invention is a cell collecting system including: the aforementioned cell collection apparatus; and an observation apparatus that observes a section on the substrate.

The fourth aspect of the present invention is a tissue displaying system including: the aforementioned cell collection apparatus; an observation apparatus that observes the section on the substrate; and a displaying unit that displays the section observed by the observation apparatus.

The fifth aspect of the present invention is a substrate configured to have a surface to which a section of a biological tissue can be pasted, be dividable into a plurality of small pieces, and divide the section pasted on the surface by being divided into the plurality of small pieces.

The sixth aspect of the present invention is an expandable member configured to: be adhered to an opposite surface to a surface to which a section of a biological tissue can be pasted of a substrate dividable into a plurality of small pieces; be expandable in a surface direction of the substrate; and expand in the surface direction so as to divide the substrate where the expandable member is adhered into the plurality of small pieces.

The seventh aspect of the present invention is a tissue dividing method including: a pasting step of pasting a section of a biological tissue on a substrate which has a surface to which the section of the biological tissue can be pasted, and is dividable into a plurality of small pieces in such a manner that the section is pasted on the surface of the substrate across the plurality of small pieces; and a dividing step of dividing the section pasted on the surface of the substrate by dividing the substrate into the plurality of small pieces.

The eighth aspect of the present invention is a cell collecting method including: a pasting step of pasting a section of a biological tissue on a substrate having a surface to which the section can be pasted, and dividable into a plurality of small pieces; a dividing step of dividing the substrate to which the section is pasted into the plurality of small pieces, to thereby divide the section into fragments each having a substantially equivalent shape to a shape of each small piece; and a pickup step of picking up the fragments of the divided section.

### {Advantageous Effect of Invention}

According to the present invention, it is possible to provide an effect to divide a section of a biological tissue into fragments in a size of including a sufficient amount of cells with a simple structure and a simple operation.

### {Brief Description of Drawings}

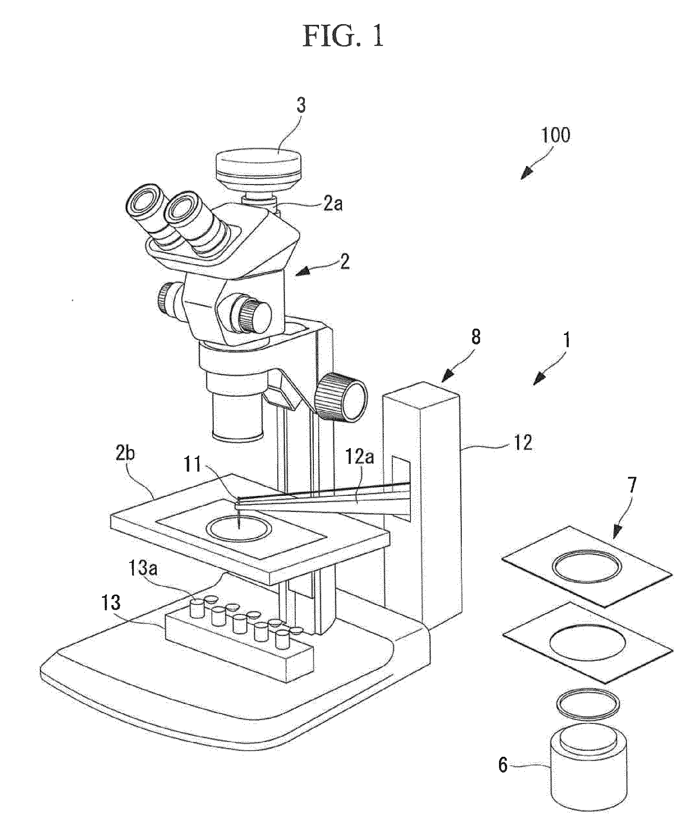

{Fig. 1}
   Fig. 1 shows the overall structure of the cell collecting system according to the first embodiment of the present invention.
{Fig. 2A}
   Fig. 2A is a side view of the glass substrate adhered to the sticky sheet, where the grooves are faced to the sticky sheet side.
{Fig. 2B}
   Fig. 2B is a side view of the glass substrate adhered to the sticky sheet, where the grooves are faced to the opposite side against the sticky sheet.
{Fig. 3A}
   Fig. 3A shows the glass substrate adhered to the sticky sheet.
{Fig. 3B}
   Fig. 3B shows the glass substrate divided by expanding the sticky sheet.
{Fig. 4}
   Fig. 4 shows the structure of the expanding stage and the jig.
{Fig. 5A}
   Fig. 5A is a diagram explaining the method how to use the expanding stage and the jig of Fig. 4, and showing a state where the sticky sheet has been expanded.
{Fig. 5B}
   Fig. 5B is a diagram explaining the method how to use the expanding stage and the jig of Fig. 4, and showing a state where the sticky sheet is held by the grip rings.
{Fig. 6}
   Fig. 6 is a flowchart explaining the procedure to pick up cells from the section with use of the cell collection apparatus and the cell collecting system of Fig. 1.
{Fig. 7}
   Fig. 7 shows a modified example of the substrate.
{Fig. 8}
   Fig. 8 shows another modified example of the substrate.
{Fig. 9}
   Fig. 9 shows a modified example of the cell collecting system of Fig. 1.
{Fig. 10}
   Fig. 10 shows another modified example of the cell collecting system of Fig. 1.
{Fig. 11}
   Fig. 11 is a flowchart showing a modified example the procedure to pick up cells with use of the cell collecting system of Fig. 1.
{Fig. 12}
   Fig. 12 is a diagram showing a modified example of the method to expand the sticky sheet with use of the outer cylinder and the inner cylinder, and showing a state where the sticky sheet is set on the outer cylinder.
{Fig. 13}
   Fig. 13 shows a state where the sticky sheet is being expanded by using the outer cylinder and the inner cylinder of Fig. 12.
{Fig. 14}
   Fig. 14 shows a state where the sticky sheet is being expanded by another method using the outer cylinder and the inner cylinder of Fig. 12.
{Fig. 15A}
   Fig. 15A shows another modified example of the method to expand the sticky sheet before expanding the sticky sheet.
{Fig. 15B}
   Fig. 15B shows the another modified example of the method to expand the sticky sheet after expanding the sticky sheet.
{Fig. 16}
   Fig. 16 shows a modified example of the grip rings.
{Fig. 17}
   Fig. 17 shows a configuration of a substrate included in a cell collection apparatus and a cell collecting system according to the second embodiment of the present invention.
{Fig. 18}
   Fig. 18 shows a configuration of a support member and a dividing auxiliary unit included in the cell collection apparatus and the cell collecting system according to the second embodiment of the present invention.
{Fig. 19A}
   Fig. 19A is a diagram explaining a dividing method of a section using the support member and the dividing auxiliary unit of Fig. 18.
{Fig. 19B}
   Fig. 19B is a diagram explaining the dividing method of the section using the support member and the dividing auxiliary unit of Fig. 18.
{Fig. 20}
   Fig. 20 shows a modification of the dividing method of the section using the support member of Fig. 18.
{Fig. 21}
   Fig. 21 is a diagram explaining a modification of the support member and the dividing method of the section using this support member.
{Fig. 22}
   Fig. 22 shows a modification of the support member of Fig. 18.
{Fig. 23}
   Fig. 23 is a photograph of the section made by the Example of the present invention, showing a state before the section has been divided.
{Fig. 24}
   Fig. 24 is a photograph showing a state after the section of Fig. 23 has been divided.

### {Description of Embodiments}

### (First Embodiment)

Hereunder is a description of a cell collection apparatus 1 and a cell collecting system 100 according to a first embodiment of the present invention with reference to Figs. 1 to 16.

As shown in Fig. 1, the cell collecting system 100 of this embodiment comprises an optical microscope (observation device) 2 and the cell collection apparatus 1 of this embodiment.

The optical microscope 2 is of an erecting type. The optical microscope 2 is configured such that an imaging device 3 like a CCD camera for taking an image of the field of view is connected to a camera port 2a. The image taken by the imaging device 3 is displayed on a monitor (not shown). The cell collecting system 100 along with this monitor constitutes a tissue displaying system. The image may be subject to image processing with an image processing device (not shown) like an image processor, and thereafter displayed on the monitor.

The cell collection apparatus 1 includes: a glass substrate (substrate) 4 to which a section of a biological tissue is to be pasted; a sticky sheet (expandable member) 5 to which the glass substrate 4 is adhered, and which is expandable in directions along the surface; an expanding stage (dividing unit, expansion unit, pressing member) 6 for expanding the sticky sheet 5; a jig 7 for holding the sticky sheet 5 while the sheet 5 is being expanded by the expanding stage 6; and a pickup unit 8 for picking up small pieces 4b of the glass substrate 4 from the sticky sheet 5. A cell dividing apparatus according to the present invention is corresponding to a configuration including the substrate 4, the expanding stage 6, and the jig 7.

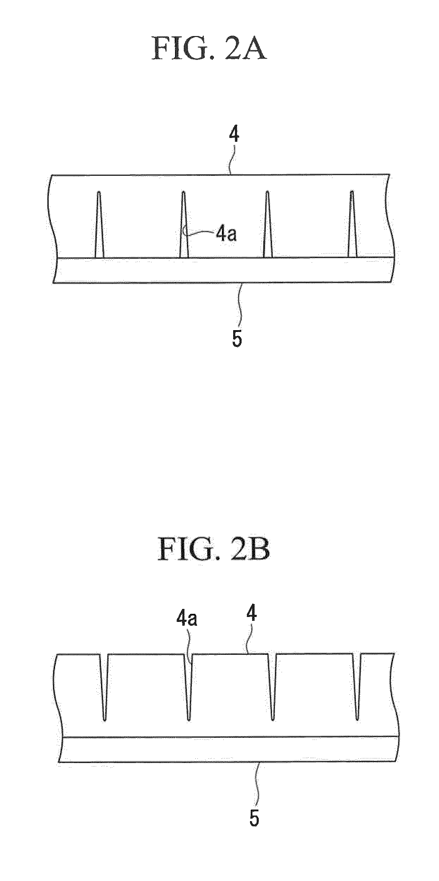

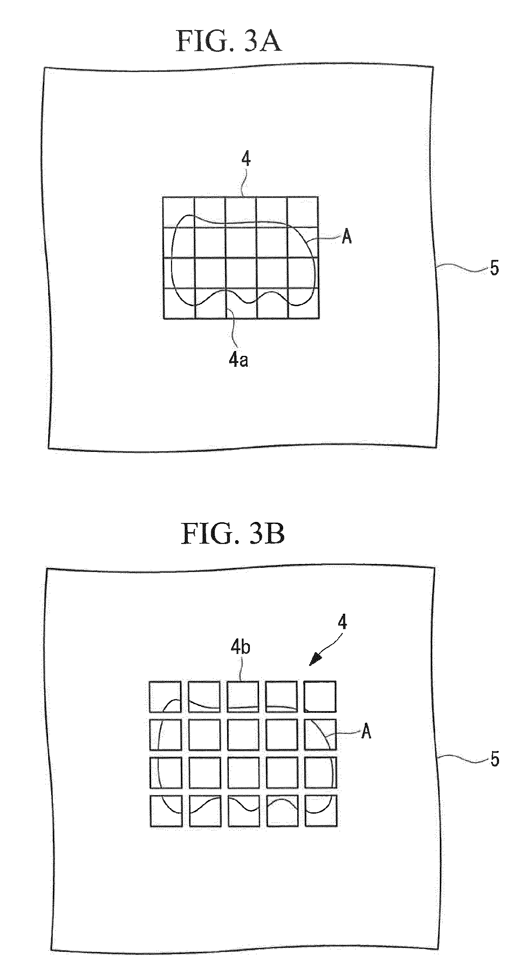

The glass substrate 4 is shaped like a plate having a flat surface. As shown in Fig. 2A and Fig. 3A, the surface of the glass substrate 4 is formed with grid-like grooves (dividing lines) 4a with predetermined spacing. The grooves 4a can be formed by, for example, laser processing, chemical etching, dicing, or the like. It is also possible to form them by hand work of an operator using a glass cutter or the like.

Preferably, the glass substrate 4 has a thickness which enables to stably support a section of a biological tissue, for example, from 0.05 mm to 0.5 mm. The spacing between the grooves 4a can be modified appropriately according to the size of fragments to be picked up from the section. For example, the spacing between the grooves 4a is preferably from 0.05 mm to 5.0 mm so that a sufficient amount of cells can be contained in the picked up fragments while the fragments of a desired region can be picked up from the section with adequately precise positional accuracy. An optically transparent substrate is used for the glass substrate 4, so that the transmitted beam image of the section pasted thereon can be observed with the optical microscope 2.

It is also possible to apply the glass substrate 4 with silanization or such a chemical treatment on the surface so as to improve the adhesiveness between the surface and the section. Also, it is possible to apply, for example, a coating treatment for preventing adsorption of nucleic acids, proteins, and the like to the glass substrate 4, according to the purpose of the test to be conducted after picking up the small pieces 4b.

One surface of the sticky sheet 5 is coated with an adhesive agent to have a stickiness which enables the glass substrate 4 to be sufficiently and firmly adhered thereto, as well as enabling it to be detached therefrom by a pickup unit 8 that will be described later. In addition, the sticky sheet 5 is expandable in directions along the surface. When the sticky sheet 5 is expanded in a direction along the surface in a state where the glass substrate 4 is being adhered onto the sticky sheet 5, the glass substrate 4 is also extended in the direction along the surface together with the expanding of the sticky sheet 5. As shown in Fig. 3B, the glass substrate 4 is cut along the positions of the grooves 4a and divided into a plurality of small pieces 4b. At this time, the section A having being pasted on the surface of the glass substrate 4 is also extended in the direction along the surface, and thus cut along the grooves 4a.

The sticky sheet 5 is optically permeable or semipermeable, by which an object arranged on the opposite side to the object lens of the optical microscope 2 can be observed across the sticky sheet 5, at the time when observing the sticky sheet 5 with the optical microscope 2. As for the sticky sheet 5, it is possible to suitably use a dicing tape for use in temporal fixation of a wafer during a dicing step in a semiconductor production process, for example.

Note that Fig. 2A shows an example in which the glass substrate 4 is adhered to the sticky sheet 5 while the grooves 4a are facing to the sticky sheet 5 side. However, the structure may also be such that as shown in Fig. 2B the glass substrate 4 is adhered to the sticky sheet 5 while the grooves 4a are facing to the opposite side against the sticky sheet 5.

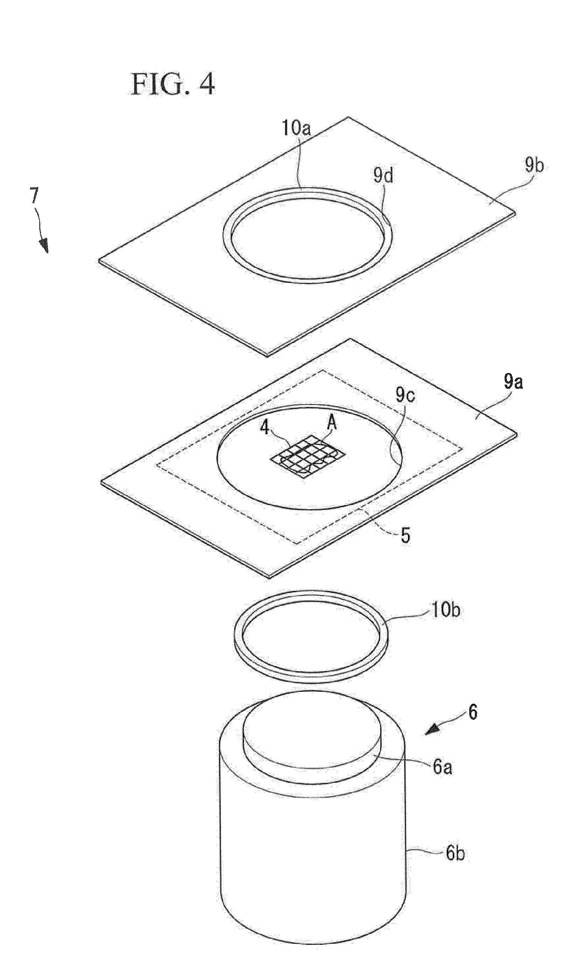

As shown in Fig. 4, the expanding stage 6 is formed to have an approximately columnar shape with approximately parallel and flat opposite ends. In the expanding stage 6, a step is formed on the lateral surface so that the upper end portion (hereunder, referred to as the small diameter portion) 6a has a diameter smaller than that of the other portion (hereunder, referred to as the large diameter portion) 6b.

The jig 7 has a bottom frame (fixing member) 9a to which the sticky sheet 5 can be pasted in an extended state, a top frame 9b for covering the bottom frame 9a from the top, and grip rings 10a and 10b (holding member) for holding the sticky sheet 5 in a expanded state. The grip rings 10a and 10b consist of an outer ring 10a and an inner ring 10b. The inner diameter of the outer ring 10a and the outer diameter of the inner ring 10b are formed to be approximately the same. In approximately the center of the bottom frame 9a and the top frame 9b, windows 9c and 9d passing through in the plate thickness direction are respectively formed. The outer ring 10a is fit inside the window 9d of the top frame 9b, along the inner circumferential surface thereof. The window 9c of the bottom frame 9a has the same or larger diameter than the outer diameter of the small diameter portion 6a.

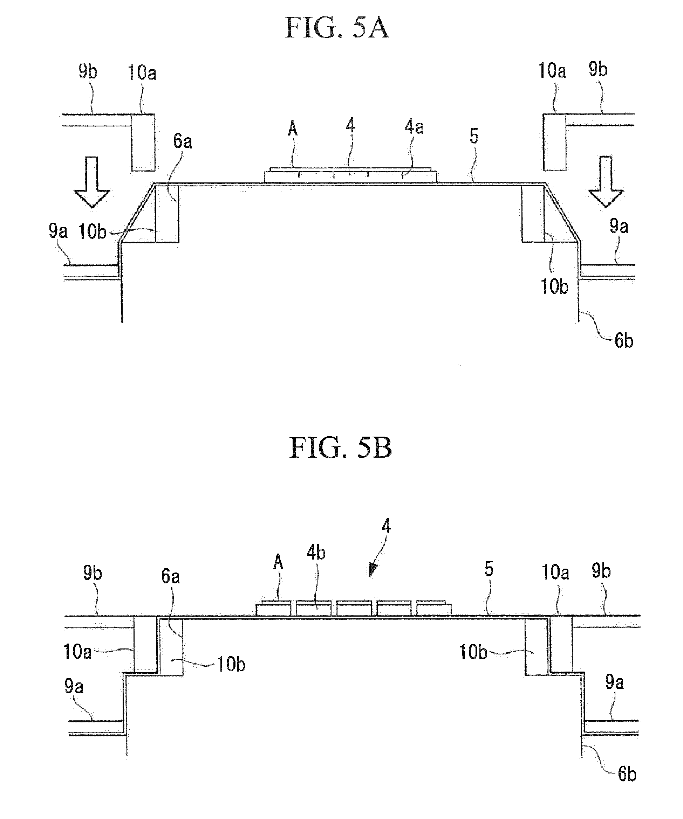

The jig 7 is used in the following manner. Firstly, the inner ring 10b is fit along the outer circumference of the small diameter portion 6a. Then, the sticky sheet 5 adhered with the glass substrate 4 is adhered to the bottom surface of the bottom frame 9a in a state where the glass substrate 4 is arranged in a position of the window 9c and the surface on the side adhered with the glass substrate 4 is facing upward. By so doing, the periphery around the region adhered with the glass substrate 4 of the sticky sheet 5 is fixed in a predetermined shape. Next, the bottom frame 9a is pressed to move downward in a state where a portion of the sticky sheet 5 corresponding to the window 9c is being pressed against the top surface of the expanding stage 6. By so doing, as shown in Fig. 5A, the portion of the sticky sheet 5 corresponding to the window 9c is expanded by being pressed from the expanding stage 6. The glass substrate 4 is divided into a plurality of small pieces 4b together with the expanding of the sticky sheet 5.

Next, as shown in Fig. 5B, the outer ring 10a and the inner ring 10b are fit to each other by covering the top frame 9b over the bottom frame 9a in a state where the sticky sheet 5 is being expanded. By so doing, the portion of the sticky sheet 5 corresponding to the window 9c is held in an expanded state by the grip rings 10a and 10b.

It is also possible to put a mark, for example, a scale mark, on the sticky sheet 5 for the purpose of understanding the degree of how much the sticky sheet 5 is expanded when the sticky sheet 5 is being expanded in this manner.

The pickup unit 8 comprises a pickup needle 11 and a manipulator 12 for operating the pickup needle 11. The tip of the pickup needle 11 has a diameter approximately the same as or smaller than the size of each small piece 4b. The manipulator 12 holds the pickup needle 11 at the distal end of the arm 12a. The manipulator 12 is designed to move the arm 12a in three dimensional directions by the operation of the operator.

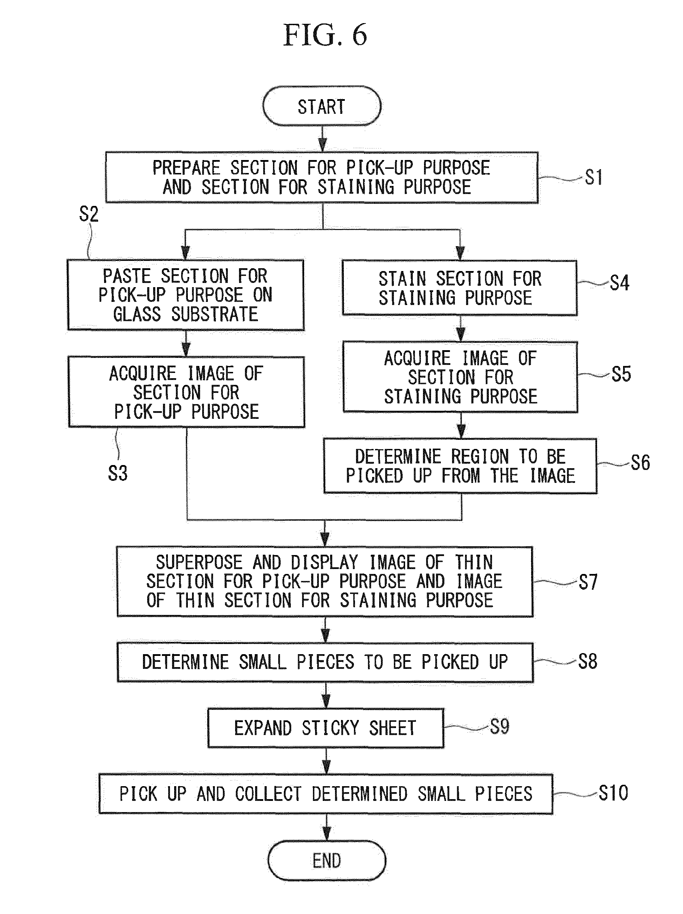

Hereunder is a description of the method to pick up fragments containing cells of interest from a section of a biological tissue by using the thus configured cell collection apparatus 1 and cell collecting system 100, with reference to Fig. 6.

In order to pick up the target cells from a section of a biological tissue by using the cell collection apparatus 1 and the cell collecting system 100 according to this embodiment: firstly, the section for pick-up purpose (section) is cut out from the biological tissue (Step S1); and the thus cut out section for pick-up purpose is pasted on the glass substrate 4 (Step S2, Pasting Step). Then, the analyte section image is acquired by observing the section for pick-up purpose with the optical microscope 2 (Step S3).

The section for pick-up purpose is cut out by, for example, freeze-embedding or paraffin-embedding method. The thickness of the section for pick-up purpose may be thicker than the thickness of a usual section for use as a pathological sample or the like (about several micrometers to ten micrometers), although it depends on the purpose of the application of the picked up cells. For example, the section for pick-up purpose can be cut out to have a thickness of about 50 µm. If the size of the thus cut out section for pick-up purpose is smaller than the spacing between the grooves 4a of the glass substrate 4, the section for pick-up purpose is pasted on the glass substrate 4 so that the section for pick-up purpose can be bridged over the groove 4a.

Moreover, differently from the section for pick-up purpose, a section for staining purpose is cut to have a thickness of about several micrometers to ten micrometers from the place adjacent to the place where the section for pick-up purpose of the biological tissue has been cut out. The thus cut out section for staining purpose is pasted on a slide glass and then stained with, for example, a dye for pathological diagnosis (Step S4). Then, the stained-section image is acquired by observing the stained section for staining purpose with the optical microscope 2 (Step S5). In the acquired stained-section image, the region to be picked up where the cells of interest exist, for example, a region infiltrated by cancer cells, is determined (Step S6).

Next, the acquired analyte section image and the stained-section image are superposed and displayed so that the sections in the respective images can exactly overlap each other (Step S7), and the positions of the small pieces 4b corresponding to the determined region to be picked up in the section for staining purpose, for example, the column numbers and the row numbers of the small pieces 4b, are recorded (Step S8).

Next, the glass substrate 4 is adhered to the sticky sheet 5. It is also possible to paste the cut out section for pick-up purpose, onto the glass substrate 4 that has been previously adhered to the sticky sheet 5. Next, the sticky sheet 5 is expanded by using the expanding stage 6 and the jig 7, to thereby divide the glass substrate 4 (Step S9, Dividing Step). At this time, the section for pick-up purpose pasted on the glass substrate 4 is also extended together with the glass substrate 4 in the surface direction, by which the section is divided into fragments along the positions of the grooves 4a.

Next, the sticky sheet 5 is held in a expanded state by the grip rings 10a and 10b, and the sticky sheet 5 is set on the specimen stage 2b so that the surface on the side adhered with the glass substrate 4 is faced downward. At this time, it is either possible to set the sticky sheet 5 on the specimen stage 2b in a state detached from the bottom frame 9a, or to set the sticky sheet 5 on the specimen stage 2b together with the bottom frame 9a.

Next, while observing the small pieces 4b in the recorded positions with the optical microscope 2, the manipulator 12 is operated within the field of view thereof to arrange the pickup needle 11 on the back of a small piece 4b to be picked up. The small piece 4b to be picked up is pushed at its surface adhered to the sticky sheet 5 with the pickup needle 11 to thereby detach and drop the small piece 4b from the sticky sheet 5 (Step S10, Pickup Step). At this time, it is also possible to arrange a tube rack 13 under the specimen stage 2b so that collection tubes 13a can be previously arranged in the positions where the small pieces 4b would drop.

Throughout the above-mentioned procedure, fragments of the region where the cells of interest exist can be picked up from the section for pick-up purpose, together with the small pieces 4b. The picked up cells are used in, for example, a genetic test or the like.

In this way, according to this embodiment, the advantage is that the region where the cells of interest exist can be selectively picked up from the section with a simple structure and a simple operation only. In addition, since there is no need of an expensive structure such as a UV laser light source, another advantage is that the production cost can be kept low. Moreover, unlike the LMD, the section is mechanically cut. Thus, a section having a relatively thick thickness can be easily cut, and also the sizes of the small pieces 4b can be easily enlarged only by adjusting the spacing between the grooves 4a. Accordingly, the operation that has so far required a large amount of labor because fragments have to be picked up from a plurality of sections so as to collect a sufficient number of cells, can be sufficed only by picking up one or a small number of small pieces 4b. Thus, the advantage is that the labor and the time required for the operation can be remarkably reduced.

In the above-mentioned embodiment, the glass substrate 4 formed with the grid-like grooves 4a is used as the substrate for pasting the section. However, instead of this, it is also possible to use a glass substrate 4 comprising glass-made small pieces 4b aligned in the planar direction.

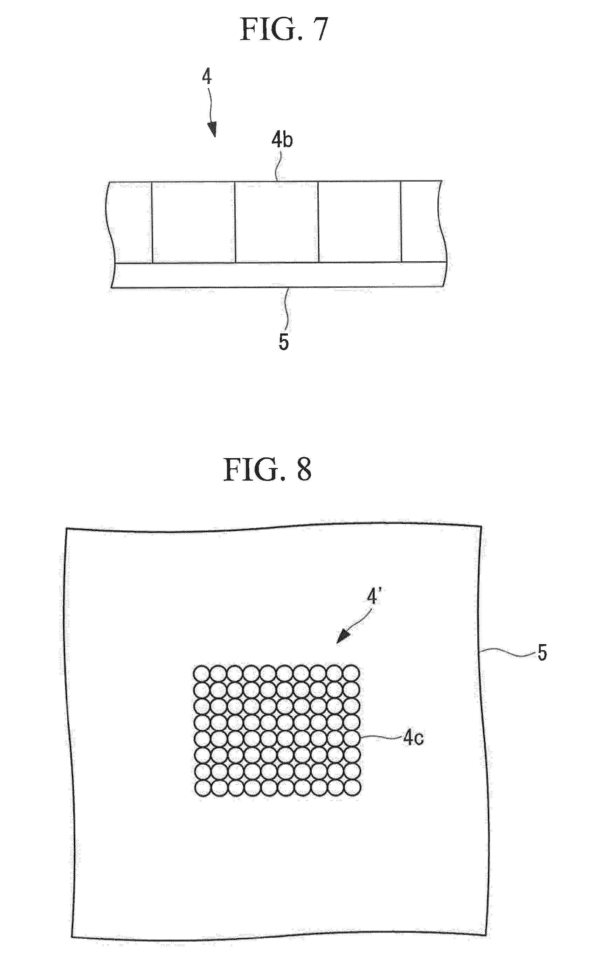

In this case, in order to keep the shape of the glass substrate 4, the glass-made small pieces 4b are previously adhered onto the sticky sheet 5 in an aligned state as shown in Fig. 7. By so doing, the glass substrate 4 can be more easily and reliably divided along the border (dividing lines) between the small pieces 4b when the sticky sheet 5 is expanded.

The following method can be adopted as an example to produce the aligned state of the previously divided small pieces on the sticky sheet 5.

The glass substrate 4 is adhered on the sticky sheet 5, and the glass substrate 4 is scratched by a glass cutter. Then, the glass substrate 4 is cracked along the position of the scratch by hand. By so doing, the aligned state of the small pieces 4b on the sticky sheet 5 as shown in Fig. 7 can be easily produced. At this time, it is preferable to carry out this procedure while protecting the surface of the glass substrate 4 with a film or the like so as to avoid contamination of the glass substrate 4.

In addition, the structure of the substrate employed in the above-mentioned embodiment is only an example, and the present invention is not to be limited to this structure.

For example, the material of the substrate may be not only a glass but also a resin or the like. Regarding the shape of the small piece 4b, any shape may be adopted as long as the small pieces 4b can be densely aligned on the surface of the sticky sheet 5. For example, a regular hexagonal shape or a triangular shape may be adopted. Moreover, as shown in Fig. 8, it is also possible to configure the substrate 4' by using globular glass beads or resin beads 4c as the small pieces and aligning them on the sticky sheet 5, preferably in a single layer.

In addition, a material having a magnetic property may also be used as the substrate. For example, it is possible to densely align magnetic particles having a diameter of 1 µm to 500 µm on the surface of the sticky sheet 5, and to use a metal plate of such as a stainless-steel as the substrate. If a material such as a metal which does not allow the transmission of visible light is used as the substrate, the small pieces 4b, even though these are small, can be clearly observed by eyes at the time when observing the transmitted beam image of the sticky sheet 5 with the optical microscope.

In this case, a magnet can be used as the pickup unit 8. For example, a magnet having approximately the same size as that of the magnetic particle is provided at the tip of the pickup needle 11. Then, the tip of the pickup needle 11 is brought closer to a magnetic particle from the opposite side against the sticky sheet 5, by which a desired magnetic particle can be held on the tip of the pickup needle 11 and picked up from the sticky sheet 5 by the magnetic force.

Moreover, in the above-mentioned embodiment, the employed structure is such that the optical microscope 2 of an erecting type is used and the expanding stage 6 is provided separately from the optical microscope 2. However, the structure of the cell collecting system 100 is not to be limited to this. For example, an inverted type can be adopted as the optical microscope 2, and the expanding stage 6 can be installed in the optical microscope 2.

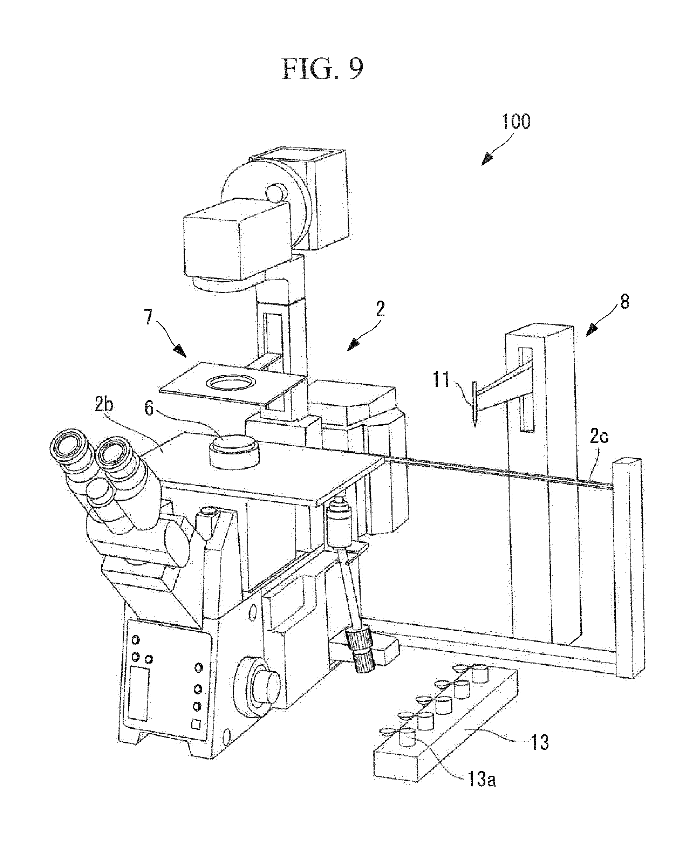

Fig. 9 shows an example of the structure in which the expanding stage 6 and the jig 7 are provided to an inverted-type optical microscope 2. The specimen stage 2b is provided slidably in the horizontal direction along a guide rail 2c bridged between the observation position of the optical microscope 2 and a position out of the main body of the optical microscope 2. In such a structure, the sticky sheet 5 is expanded on the expanding stage 6 by moving the jig 7 downward in a state where the specimen stage 2b is arranged in the observation position. Thereafter, the specimen stage 2b is slid and the small pieces 4b are picked up by using the pickup unit 8 having been provided separately from the optical microscope 2.

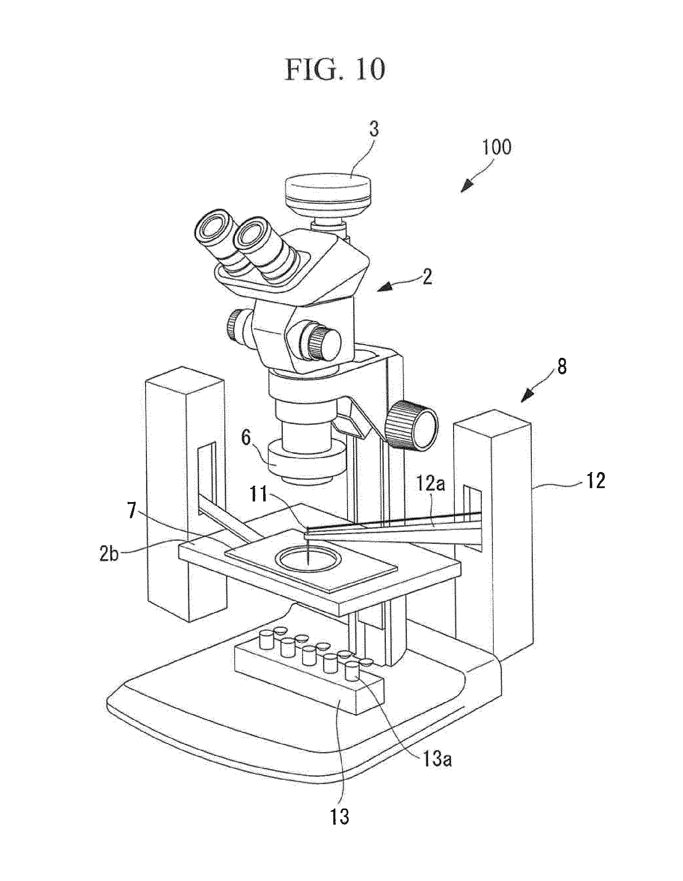

Fig. 10 shows an example of the structure in which the expanding stage 6, the jig 7, and the pickup unit 8 are provided to an inverted-type optical microscope 2. The expanding stage 6 is provided above the specimen stage 2b so that the stage can be moved integrally with the object lens in the vertical direction, and the expanding stage 6 is designed to be pushed against the sticky sheet 5 held on the specimen stage 2b by the jig 7, from the above. In this structure, a series of operations from the expanding of the sticky sheet 5 to the pickup of the small pieces 4b can be done on the specimen stage 2b.

Moreover, in the above-mentioned embodiment, the small pieces 4b to be picked up are determined by making a comparison between the analyte section image of the section for pick-up purpose and the stained-section image of the section for staining purpose. However, instead of this, it is also possible to determine the small pieces 4b to be picked up from the image of the section for pick-up purpose.

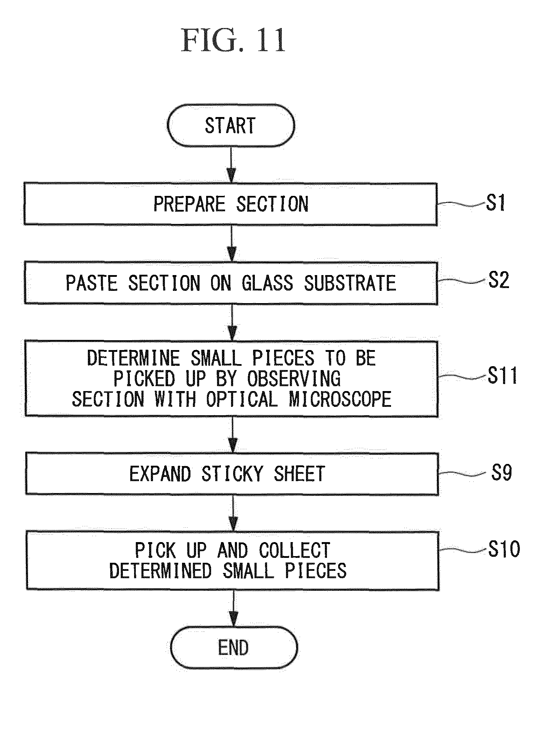

In the case where a section for pick-up purpose has a relatively thick thickness, cells are overlapped in the thickness direction. This makes it difficult to accurately recognize the shape of each cell, the distribution of cells, and the like, from the image, even though the image is stained. However, even from such a stained-section image or an unstained-section image of the section, in the case where the determination of the region to be picked up is possible based on a rough shape of the tissue or on the distribution of cells, for example as shown in Fig. 11, the small pieces 4b to be picked up can be selected using the same image while observing the section for pick-up purpose with the optical microscope 2 (Step S11). Thereafter, the sticky sheet 5 can be expanded (Step S9) and the small pieces 4b can be picked up (Step S10). By so doing, the procedure can be much more simplified as required.

Moreover, in the above-mentioned embodiment, the approximately columnar expanding stage 6 is used to expand the sticky sheet 5. However, the unit to expand the sticky sheet 5 is not to be limited to this.

For example, instead of the expanding stage 6, an outer cylinder 14a and an inner cylinder 14b that can be accommodated in the outer cylinder 14a can be adopted.

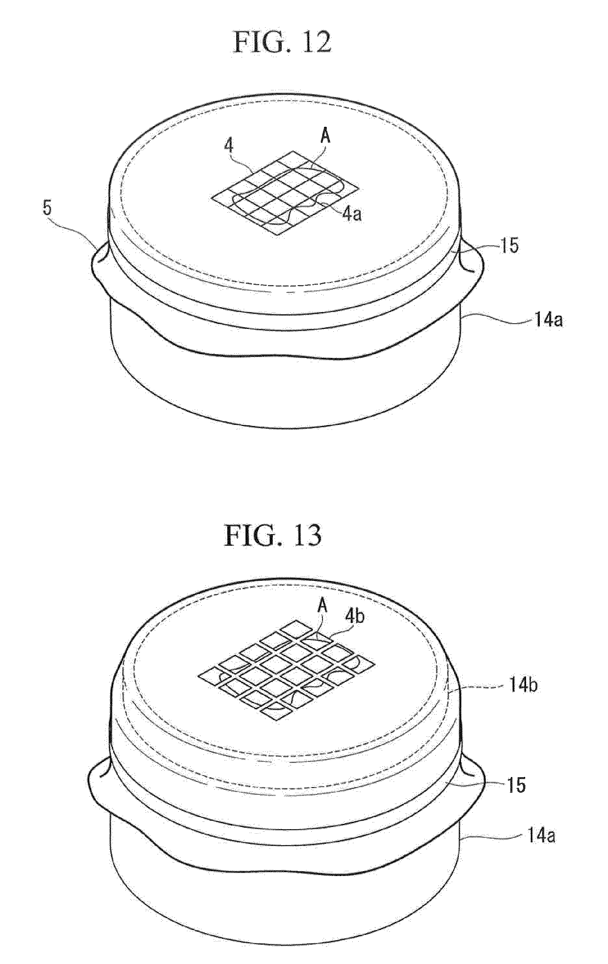

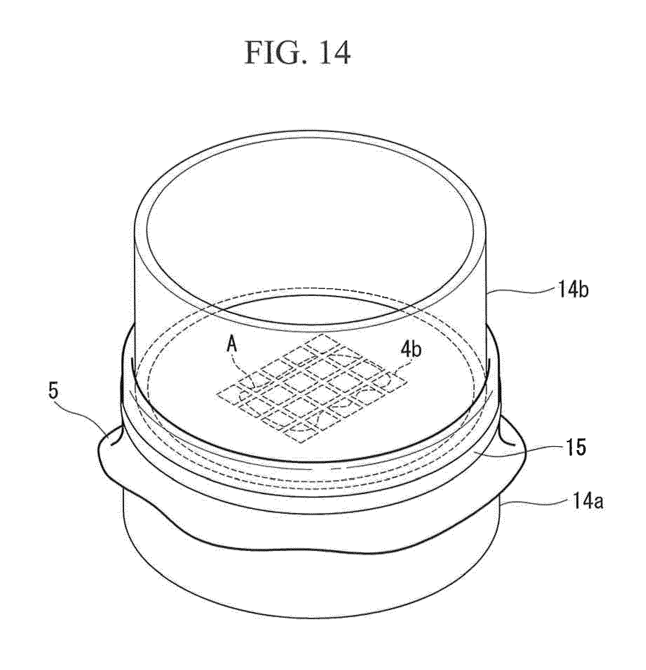

In this case, as shown in Fig. 12, the sticky sheet 5 is covered over the top surface of the outer cylinder 14a and fixed to the lateral surface of the outer cylinder 14a. The symbol 15 denotes a fixing member for fixing the sticky sheet 5 to the lateral surface of the outer cylinder 14a. Next, as shown in Fig. 13 the inner cylinder 14b is protruded relatively from the inside of the outer cylinder 14a, or as shown in Fig. 14 the inner cylinder 14b is pushed relatively from the outside of the outer cylinder 14a into the inside of the outer cylinder 14a. By so doing, the sticky sheet 5 can be expanded approximately evenly in every direction.

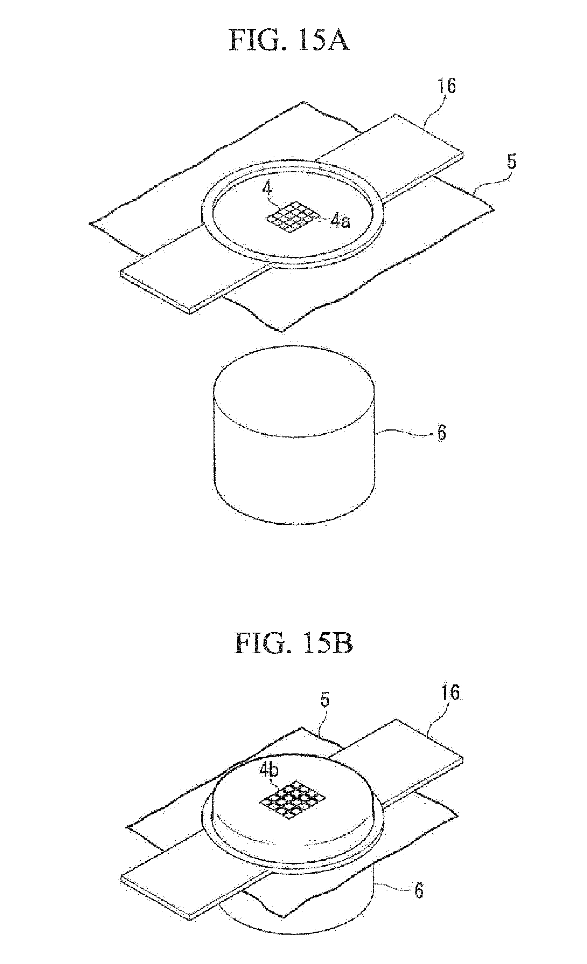

In addition, the structure may also be such that as shown in Fig. 15A the sticky sheet 5 is held in a certain shape by the holder 16 in a position where the region adhered with the glass substrate 4 is excluded, and as shown in Fig. 15B the sticky sheet 5 is expanded by pressing the region adhered with the glass substrate 4 against the expanding stage 6.

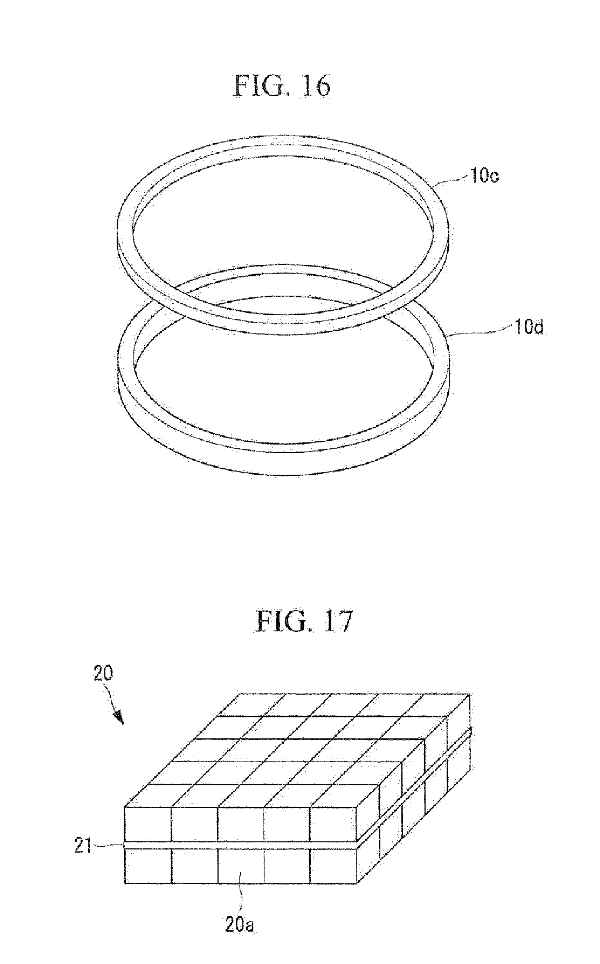

Moreover, as for the grip rings, it is also possible to adopt rings that can be held in a mutually and tightly attached state by a magnetic force. For example, as shown in Fig. 16, grip rings 10c, 10d may be composed of a top ring 10c and a bottom ring 10d both having the same diameters, and the top ring 10c is made of a magnet and the bottom ring 10d is made of a metal, such as stainless steel. Both the top ring 10c and the bottom ring 10d can be tightly attached to each other by a magnetic force while holding the sticky sheet 5 in an expanded state between the top ring 10c and the bottom ring 10d, to thereby hold the sticky sheet 5 in an expanded state. The both rings 10c and 10d may also be composed of magnets which generate attracting forces each other.

Moreover, in the above-mentioned embodiment, a small piece 4b is detached and collected from the sticky sheet 5 by pushing the small piece 4b from the back with the pickup needle 11. However, instead of this, it is also possible to use a collecting container the interior of which can be sucked to a negative pressure. By so doing, a small piece 4b to be picked up can be detached from the sticky sheet 5 and collected into the collecting container by bringing the collecting container closer to the small piece 4b from the opposite side against the sticky sheet 5.

Moreover, a substance that loses the stickiness by UV irradiation can also be used as the sticky sheet 5.

In this case, for example, a UV light source (pickup unit) is equipped in the optical microscope 2. Also, UV light radiated from the UV light source is irradiated locally to the position of a small piece 4b to be picked up through the object lens, by which the small piece 4b can be easily detached and collected from the sticky sheet 5. Besides, a similar effect can also be achieved by guiding UV light through an optical fiber and locally irradiating the light toward the position of the small piece 4b to be picked up while orienting the fiber end thereto.

### (Second Embodiment)

The cell collection apparatus and the cell collecting system according to the second embodiment of the present invention will be described with reference to Fig. 17 to Fig. 22, hereinafter.

The cell collection apparatus and the cell collecting system according to the present embodiment are different in the configurations of the substrate, the dividing unit, and the pickup unit from those in the aforementioned first embodiment. Hence, the configurations of these components will mainly be described, and the description of the same configurations as those in the first embodiment will be omitted.

In the present embodiment, the substrate 20 is formed of an assembly of a plurality of small pieces 20a two-dimensionally arranged in a planar state, and as shown in Fig. 17, the small pieces 20a in a substantially cuboid shape are held with the side surfaces thereof in tight contact with one another. At least one of the end surfaces of the small pieces 20a is arranged in an identical plane which forms a substantially flat surface to which a section for pick-up purpose of a biological tissue (referred to simply as a section, hereinafter in the present embodiment) can be pasted. Hereinafter, each surface of the substrate 20 and the small pieces 20a on which the section is pasted is also referred to as a tissue pasting surface.

The above arrangement structure of the small pieces 20a is maintained by binding them into one piece with an annular member 21 that inwardly exerts a pushing force in the radial direction, such as a rubber band, for example. To be specific, the adjacent small pieces 20a are pressed at the side surfaces thereof to one another so that the arrangement structure of the small pieces 20a is maintained by a friction force generated at the side surfaces of the adjacent small pieces 20a. In this arrangement structure, each small piece 20a is supported by the plural adjacent small pieces 20a of this small piece 20a, and thus even if one of the small pieces 20a is removed, the arrangement structure of the remaining small pieces 20a is stably maintained.

The small pieces 20a may be maintained in the arrangement state by bonding the adjacent side surfaces thereof to one another with adhesive, or by applying adhesive on opposite surfaces to the tissue pasting surfaces thereof. It is preferable that the substrate 20 is made of such a material that is easily microfabricated, has a surface with a preferable stickiness to the section, and is made of a material free from contaminant of biological materials, such as a material made of glass, metal, and plastics, etc., for example. Each small piece 20a may have any shape as far as the arrangement structure can be maintained, and may have a global, cylindrical, or polygonal shape other than a cuboid shape. The small piece 20a in a regular hexagonal prism shape attains a more stable arrangement structure.

In the present embodiment, the small piece 20a indicates a single small piece in a cuboid shape, and the small pieces 20a indicate a group of small pieces excluding this small piece.

The cell collection apparatus according to the present embodiment includes a support member 22 and a dividing auxiliary unit 23 instead of the sticky sheet 5, the expanding stage 6, and the jig 7.

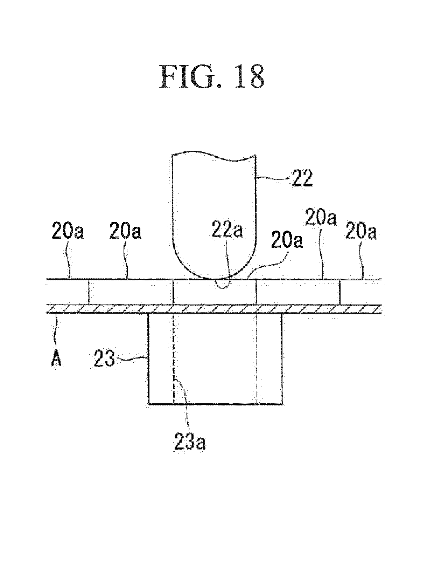

The support member 22 has a contact surface 22a to be in contact with the end surface of a single small piece 20a with a smaller area than the area of this end surface. As an example of the support member 22, a cylindrical member having an end surface in an approximately semi-spherical shape is illustrated in Fig. 18, but the contact surface 22a of the support member may have any shape as far as the contact surface 22a can stably press the end surface of the small piece 20a, and may have a substantially flat shape which secures contact with the end surface of the small piece 20a with a sufficiently large area, for example.

The dividing auxiliary unit 23 assists dividing of the section using the support member 22. The dividing auxiliary unit 23 is a cylindrical member, and has a hole 23a extending in the longitudinal direction, and opening in both end surfaces thereof. At least one of the end surfaces of the dividing auxiliary unit 23 is a flat surface substantially perpendicular to the longitudinal direction. When this end surface is disposed in contact with the end surface of the small piece 20a, the hole 23a is configured to extend in the thickness direction of the small piece 20a. The cross sectional shape of the hole 23a may be any shape having a larger size than the end surface of the small piece 20a so that the small piece 20a can pass through the hole 23a, and the hole 23a preferably has a cross sectional shape slightly larger than the end surface of the small piece 20a.

A method of picking up fragments including desired cells from a section of a biological tissue using the cell collection apparatus and the cell collecting system as configured above will be described. The tissue dividing method and the cell collecting method according to the present embodiment are different in the step of dividing the section (Step S9) and the step of picking up divided fragments (Step S10) from those of the first embodiment shown in Fig. 6. Hence, only these two steps will be described.

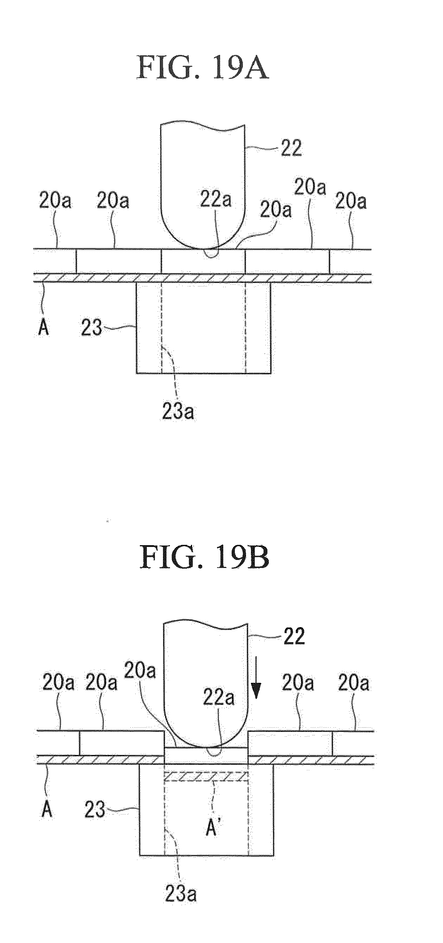

After the substrate 20 is placed on the specimen stage 2b in such a manner that the tissue pasting surface to which the section A is pasted is faced downward, the support member 22 is arranged to an opposite surface to the tissue pasting surface (also referred to as an opposite surface, hereinafter) of the small pieces 20a to be picked up, as shown in Fig. 19A. The dividing auxiliary unit 23 is arranged in such a manner that the aperture of the hole 23a opposes the support member 22 with the substrate 20 held therebetween, and one end surface of the dividing auxiliary unit 23 is brought into contact with the section A. The operation of the support member 22 is carried out by operating the manipulator 12 in the same manner as the pickup needle 11 of the first embodiment.

As shown in Fig. 19B, the support member 22 is moved downward to press the opposite surface of the small piece 20a with the contact surface 22a of the support member 22 to thereby downwardly move this small piece 20a (Dividing Step, Pickup Step). The small pieces 20a are supported by one another with a friction force; therefore, while the arrangement state of the group of the other small pieces 20a around this small piece 20a is maintained, only this small piece 20a pressed by the support member 22 is moved downward, and separated from the group of the other small pieces 20a therearound.

A part of the section A pasted on the tissue pasting surface of the separated small piece 20a is pulled down with respect to the other portion thereof to thereby cut this part of the section A into a fragment A' having substantially the same shape as that of the tissue pasting surface of this small piece 20a. At this time, an edge between one end surface of the dividing auxiliary unit 23 and the inner peripheral surface of the hole 23a works as a cutting blade relative to the section A. This configuration facilitates cutting of the section A, and enables the fragment A' to have a shape that more precisely matches the shape of the tissue pasting surface of the small piece 20a. The small piece 20a separated together with the fragment A' of the section A in this manner are dropped and collected by a collecting tube 13a prepared in advance below the specimen stage 2b.

Through the above procedure, the fragment A' in a region where the desired cells exist can be picked up together with the small piece 20a from the section A.

The present embodiment has an advantage that a region where the desired cells exist can be selectively picked up from the section A only with a simple structure and a simple operation. In addition, there is another advantage that requires no expensive configuration, such as a UV laser source, which reduces manufacturing cost. Different from the LMD, the section A is cut off in a mechanical manner, and thus this makes it easier to cut off the section A having a relatively thick thickness, and also to enlarge the size of the fragment A' simply by adjusting the size of the small piece 20a. Accordingly, different from the conventional collecting operation that requires tremendous labor of collecting fragments from plural sections in order to collect a sufficient amount of cells, it is only required to collect a single or a few of the small pieces 20a, to thereby attain such an advantage that significantly reduces labor and time spent on the collecting operation.

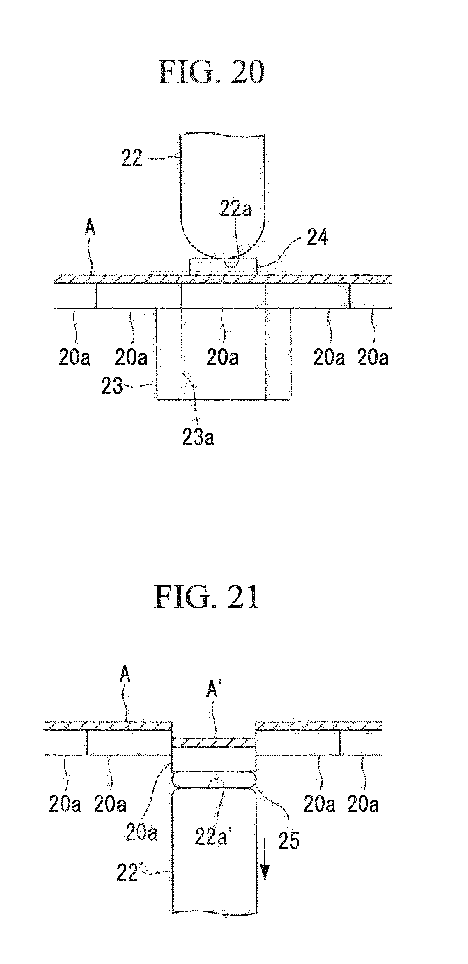

In the aforementioned embodiment, it is configured to press the opposite surface of the small piece 20a with the support member 22, but instead of this, it may be configured to press the tissue pasting surface of the small piece 20a, as shown in Fig. 20. In this case, the substrate 20 is placed on the specimen stage 2b with the surface of the substrate 20 where the section A is pasted faced upward, to thereby readily drop the small piece 20a separated by the support member 22 into the collecting tube 13a below.

If pressing the tissue pasting surface of the small piece 20a with the support member 22, a direct contact of the contact surface 22a of the support member 22 with the section A may be prevented by providing a protective member 24 therebetween.

This configuration prevents substances, such as biological molecules, contained in the section A from adhering to the support member 22; therefore, even if the fragments A' are picked up by separating the plural small pieces 20a successively using the identical support member 22, it is possible to prevent contamination among the fragments A' via the support member 22.

In this case, the protective member 24 having substantially the same size as, or smaller than that of the tissue pasting surface of the small piece 20a may be used, and after the small piece 20a is separated, the protective member 24 may be collected together with the small piece 20a. In this configuration, the protective member 24 may be formed of a material causing no influence on the analysis of the fragment A' pasted on the small piece 20a. In this manner, it is also possible to prevent contamination among the fragments A' via the support member 22.

In the case of using the section A produced with the paraffin embedding method, substances, such as biological molecules, contained in the section A are most unlikely to adhere to the support member 22 when the support member 22 comes into contact with the section A. Hence, even if the same support member 22 is used without using the protective member 24 for successively separating the small pieces 20a, it is most unlikely to cause contamination among the fragments A' picked up. In the case of using a section whose cut surface of the biological tissue is exposed like the section A produced with the freeze-embedding method, although the fragments A' to be picked up are sufficiently large so that slight contamination may be generated among them, it is unnecessary to use the protective member 24 as far as no influence is caused to the result of the subsequent analysis on the fragments A'.

In the aforementioned embodiment, instead of using the support member 22, any other means may be used for pressing the tissue pasting surface or the opposite surface of the small piece 20a. For example, it may be configured that the dividing unit includes a tubular member having a narrower aperture than the end surface of the small piece 20a, and compressed air is supplied to the inside of the tubular member while the aperture is oppositely disposed to the end surface of the small piece 20a so as to inject the compressed air from the aperture to the end surface of the small piece 20a, to thereby press this end surface.

In the aforementioned embodiment, it is configured to separate the small piece 20a by pressing the tissue pasting surface of this small piece 20a, but it may be configured to pull the opposite surface of the small piece 20a, instead.

In this case, a support member 22' is used for separating the small piece 20a. To be specific, as shown in Fig. 21, an attracting member 25 that attracts the opposite surface of the small piece 20a is disposed to a contact surface 22a' of the support member 22', and the contact surface 22a' is brought into contact with the opposite surface via the attracting member 25 so that the small piece 20a is attracted to come into contact with the support member 22'. The support member 22' is then moved in the direction indicated by the arrow so as to separate one of the small pieces 20a. The separated small piece 20a is detached and collected from the attracting member 25. Fig. 21 shows the support member 22' having the substantially flat contact surface 22a'.

A member having a sticky surface is used for the attracting member 25, for example. If the small pieces 20a contain magnetic substances, a magnet may be used as the attracting member 25. In addition, the aforementioned tubular member may be used such that its aperture is oppositely disposed to the end surface, and the inside of the tubular member is sucked so as to pull the opposite surface of the small piece 20a, to thereby separate the small piece 20a.

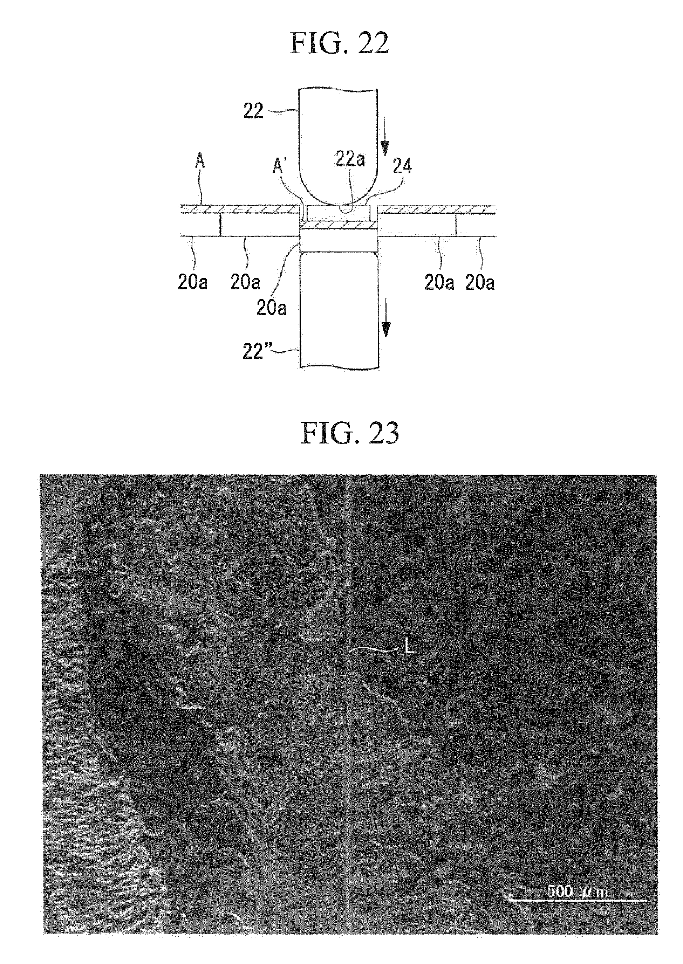

In the above described embodiment, as shown in Fig. 22, such a configuration may be employed that two support members 22, 22" are used to support the both end surfaces of the small piece 20a, and these support members 22, 22" are integrally moved upward or downward to separate the small piece 20a.

In the aforementioned embodiment, the direction of separating the small piece 20a is not limited to the upward or downward directions in the vertical direction, and the small piece 20a may be moved to be separated in the oblique direction.

### {Examples}

Next is a description of Example of the above-mentioned embodiment.

In this Example, the following experiment was conducted so as to confirm that the cell collection apparatus was capable of dividing a section of a biological tissue.

A pig colon was used as the biological tissue. A section having four side lengths of about 10 mm and a thickness of 50 µm was cut out from the biological tissue by freeze-embedding method. The cut out section was pasted on four cover glasses, which had been aligned to be adjacent to each other at the corners and pasted on a dicing sheet (expandable member), so that the section was bridged over the border (dividing line) between these cover glasses. Thereafter, the section was dried by air. The cover glass used herein had respective side lengths of 18 mm and a thickness of 0.13 to 0.17 mm. Specifically, in the present Example, one substrate is constituted by four cover glasses as the small pieces, and these four cover glasses are segmented from one another by grooves.

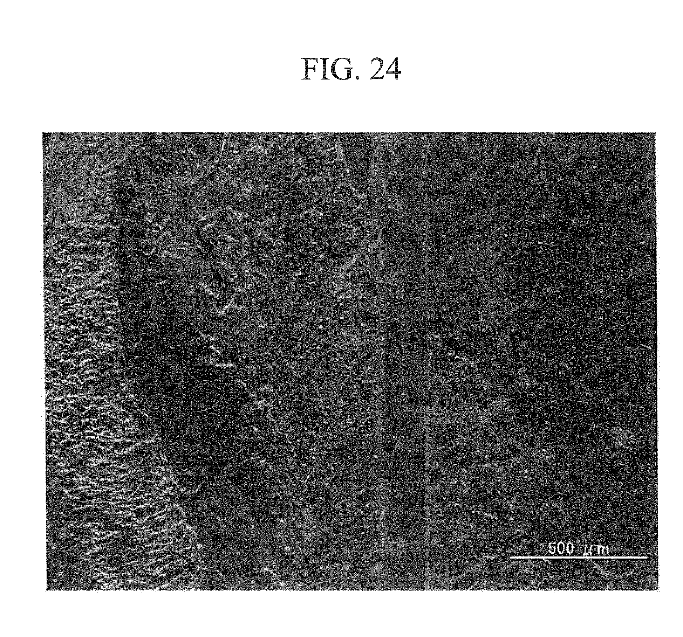

Next, the dicing tape was pasted on the top frame (fixing member) having a circular window formed through an approximate center thereof such that the section was arranged at an approximate center of the window. Next, the surface to which the cover glasses were pasted was faced upward, and in a state where the columnar stage (pressing member, expansion unit, dividing unit) was arranged in the window, the top frame was pushed down while being kept approximately horizontal, by which the portion of the dicing tape arranged within the window was expanded in the planar direction. By so doing, these four cover glasses was separated from each other, and the section pasted on the cover glasses was divided into four fragments along the border between the cover glasses. The photographs of the section before and after dividing the section in this way are shown in Fig. 23 and Fig. 24.

Fig. 23 shows a state before expanding the dicing tape. The line L seen in the vertical direction in the approximate middle of the photograph is the border (dividing line) between the cover glasses.

Fig. 24 shows the state after expanding the dicing tape. It was found that the section was sharply divided along the border between the cover glasses.

From the above-mentioned experiment, it was confirmed to be possible, by using the tissue dividing apparatus, the cell collection apparatus and the cell collecting system of the present invention, to easily divide a section sufficiently sharply along the dividing line, even though the thickness is relatively thick.

From the aforementioned first embodiment and Example, other aspects of the present invention set forth in the following appended features are introduced.

### (Feature 1)

A cell collection apparatus including: a substrate having a surface to which a section of a biological tissue can be pasted, and dividable into a plurality of small pieces; a dividing unit that divides the substrate into the plurality of small pieces, to thereby divide the section pasted on the surface of the substrate into fragments each having a substantially equivalent shape to a shape of each small piece; and a pickup unit that picks up the small pieces divided by the dividing unit.

In the invention according to feature 1, the fragments of the section can be picked up together with the small pieces by a simple configuration and a simple operation of dividing the substrate together with the section of the biological tissue pasted on the substrate with the dividing unit, and picking up the divided small pieces with the pickup unit. Increase in thickness of the section, or enlargement of each small piece in the plane direction can increase the amount of cells adhered to the small piece, and simply by picking up a single or a few of the small pieces, it is possible to easily pick up a sufficient amount of cells for a test using an extremely small amount of a biological specimen, such as gene.

### (Feature 2)

The cell collection apparatus according to feature 1, wherein the substrate is formed by arranging the plurality of small pieces in a planar state, and the dividing unit divides the substrate by applying a force to the substrate to relatively move the small pieces with respect to one another.

### (Feature 3)

The cell collection apparatus according to feature 2, wherein the substrate is provided with grooves for segmenting the substrate into the plurality of small pieces.

### (Feature 4)

The cell collection apparatus according to feature 2, wherein the substrate is formed of an assembly of the plurality of small pieces that are separated.

### (Feature 5)

The cell collection apparatus according to any one of feature 1 to feature 4, wherein the dividing unit applies a force to the substrate to relatively move the small pieces with respect to one another in directions along the surface.

### (Feature 6)

The cell collection apparatus according to feature 5, further including an expandable member adhering to an opposite surface to a surface of the substrate to which the section is pasted, and expandable in the directions along the surface, and the dividing unit including an expansion unit that expands the expandable member in the directions along the surface at least in a region included in the substrate where part of the small pieces is pasted.

In the invention according to feature 6, while the substrate to which the section is pasted is adhered onto the expandable member, the expandable member is expanded by the expansion unit, to thereby divide the section pasted on the substrate together with the substrate.

### (Feature 7)

The cell collection apparatus according to feature 6, wherein the expandable member detachably adheres to the opposite surface of substrate.

In the invention according to feature 7, it is possible to easily detach and collect the small pieces from the expandable member after the substrate is divided.

### (Feature 8)

The cell collection apparatus according to feature 7, wherein the expandable member has stickiness on its surface.

In the invention according to feature 8, the substrate can be easily adhered onto the expandable member.

### (Feature 9)

The cell collection apparatus according to feature 6 or feature 7, wherein the substrate is formed of the plurality of separated small pieces adhered to the expandable member in a mutually adjacent and aligned state.

In the invention according to feature 9, the substrate can be easily and securely divided into the small pieces when the expandable member is expanded.

### (Feature 10)

The cell collection apparatus according to any one of feature 6 to feature 9, wherein the expandable member is made of an optically transparent or semi-transparent material.

In the invention according to feature 10, the positions of the small pieces on the expandable member can be checked at the time when observing a transmitted beam image of the expandable member with an optical microscope.

### (Feature 11)

The cell collection apparatus according to any one of feature 1 to feature 10, wherein the small piece is in a cuboid shape having a thickness of 0.05 to 0.5 mm and side lengths of 0.05 to 5.0 mm.

In the invention according to feature 11, a desired region can be selectively picked up from the section with adequately precise accuracy, and also a sufficient amount of cells can be adhered to each small piece.

### (Feature 12)

The cell collection apparatus according to any one of feature 6 to feature 11, wherein the expansion unit includes: a fixing member that fixes a periphery of the region of the expandable member where the substrate is adhered in a predetermined shape; and a pressing member that presses the region of the expandable member being fixed by the fixing member, from an opposite surface to the surface where the substrate is adhered.

In the invention according to feature 12, the expandable member can be expanded only with a simple structure and a simple operation.

### (Feature 13)

The cell collection apparatus according to any one of feature 6 to feature 12, wherein the pickup unit includes a needle member that pushes a position of the expandable member where the small pieces are adhered, from the opposite surface to the surface where the small pieces are adhered.

In the invention according to feature 13, only specific small pieces among the plurality of small pieces can be easily detached and picked up from the expandable member.

### (Feature 14)

The cell collection apparatus according to any one of feature 6 to feature 13, further including a holding member that holds the above region of the expandable member in an expanded state.

In the invention according to feature 14, the expandable member can be expanded to be held in a state where the spacing between the respective small pieces is kept open, to thereby more easily pick up the small pieces with the pickup unit.

### (Feature 15)

The cell collection apparatus according to feature 14, wherein the holding member is capable of holding the expandable member with the surface of the expandable member where the substrate is adhered faced downward.

In the invention according to feature 15, the holding member can hold the expandable member with the substrate faced downward to detach the small pieces, to thereby allow the small pieces to drop by their own weights, and easily collect them.

### (Feature 16)

The cell collection apparatus according to feature 14 or feature 15, wherein a collecting container is provided to the side of the surface of the expandable member held by the holding member where the substrate is adhered, and the interior of the collecting container is sucked to a negative pressure.

In the invention according to feature 16, a small piece to be picked up can be easily collected into the collecting container only by bringing the collecting container closer to the small piece.

### (Feature 17)

The cell collection apparatus according to feature 9, wherein the small piece is a magnetic particle having a diameter of 0.001 to 0.5 mm.

In the invention according to feature 17, a treatment can be made more efficient, for example, by isolating the collected small pieces from a solution with use of the magnetic force when the small pieces are treated in the solution.

### (Feature 18)

The cell collection apparatus according to feature 17, wherein the pickup unit includes a magnet which generates a magnetic force to attract the magnetic particle in a space of an approximately same size as that of the small piece.

In the invention according to feature 18, a desired small piece can be caught by the magnet and easily picked up from the expandable member, by bringing the magnet closer to the desired small piece.

### (Feature 19)

A cell collecting system including the cell collection apparatus according to any one of feature 1 to feature 18, and an observation device that observes the section on the substrate.

In the invention according to feature 19, the selection of small pieces to be picked up and the pickup of the selected small pieces can be more easily and more accurately carried out, through the observation of the section pasted on the substrate, the small pieces formed after dividing the substrate, and the like, with the observation device.

### (Feature 20)

A cell collecting method including: a pasting step of pasting a section of a biological tissue on a surface of a substrate that can be divided into a plurality of small pieces along a predetermined dividing line, while having the section bridged over the dividing line; a dividing step of dividing the substrate and the section of the biological tissue along the dividing line, by expanding the substrate to which the section is pasted in a direction along the surface; and a pickup step of picking up the small pieces that have been divided in the dividing step.

In the invention according to feature 20, a sufficient amount of cells for a genetic test can be picked up from a section of a biological tissue with a simple structure and a simple operation by: pasting the section on the substrate, in the pasting step; thereafter dividing the section pasted on the substrate into a plurality of small pieces together with the substrate, by expanding the substrate, in the dividing step; and selectively picking up small pieces from the plurality of divided small pieces, in the pickup step.

### {Reference Signs List}

- 1: Cell collection apparatus
- 2: Optical microscope (Observation device)
- 2a: Camera port
- 2b: Specimen stage
- 3: Imaging device
- 4: Glass substrate (Substrate)
- 4a: Groove (Dividing line)
- 4b: Small piece
- 5: Sticky sheet (Expandable member)
- 6: Expanding stage (dividing unit, expansion unit, pressing member)
- 7: Jig
- 8: Pickup unit
- 9a: Bottom frame (Fixing member)
- 9b: Top frame
- 9c: and 9d Windows
- 10a: Grip ring, Outer ring (Holding member)
- 10b: Grip ring, Inner ring (Holding member)
- 10c: Grip ring, Top ring (Holding member)
- 10d: Grip ring, Bottom ring (Holding member)
- 11: Pickup needle (Needle member)
- 12: Manipulator
- 12a: Arm
- 13: Tube rack
- 13a: Collection tube
- 14a: Outer cylinder
- 14b: Inner cylinder
- 15: Fixing member
- 16: Holder
- 20: Substrate
- 20a: Small piece
- 21: Annular member
- 22: Support member
- 22a: Contact surface
- 23: Dividing auxiliary unit
- 23a: Hole
- 24: Protective member
- 25: Attracting member
- 100: Cell collecting system

## Claims

1. A tissue dividing apparatus comprising:
a substrate having a surface to which a section of a biological tissue can be pasted, and dividable into a plurality of small pieces; and
a dividing unit that divides the substrate into the plurality of small pieces, to thereby divide the section pasted on the surface into fragments each having a substantially equivalent shape to a shape of each small piece.

2. The tissue dividing apparatus according to claim 1, wherein
the substrate is formed of an assembly of the plurality of small pieces that are divided in advance,
and
the dividing unit divides the substrate by applying a force to the substrate to relatively move the small pieces with respect to one another.

3. The tissue dividing apparatus according to claim 1, wherein
the substrate is provided with grooves for segmenting the substrate into the plurality of small pieces,
and
the dividing unit divides the substrate by applying a force to the substrate to relatively move the small pieces with respect to one another.

4. The tissue dividing apparatus according to any one of claim 1 to claim 3, wherein
the dividing unit applies a force to the surface to relatively move the plurality of small pieces with respect to one another to the substrate in a crossing direction.

5. The tissue dividing apparatus according to claim 4, wherein
the dividing unit moves a part of the plurality of small pieces in the crossing direction.

6. The tissue dividing apparatus according to claim 5, wherein
the part of the small pieces comprises at least one small piece to which a region to be picked up of the section is pasted.

7. The tissue dividing apparatus according to claim 5 or claim 6, wherein
the dividing unit comprises a support member that supports at least one of surfaces of the part of the small pieces to which the section is pasted, and opposite surfaces to the surfaces, and the dividing unit moves the support member in the crossing direction and thereby moves the part of the small pieces in the crossing direction.

8. The tissue dividing apparatus according to claim 7, wherein
the dividing unit comprises a dividing auxiliary unit that assists dividing of the section pasted on the part of the small pieces.

9. The tissue dividing apparatus according to claim 8, wherein
the dividing auxiliary unit comprises a substantially cylindrical member which holds the substrate between the supporting member and the cylindrical member with an end surface of the cylindrical member opposite to the substrate, and of which end surface is provided with a hole into which the part of the small pieces moved by the support member is insertable,
and
an edge between the end surface and an inner surface of the hole assists the dividing of the section.

10. The tissue dividing apparatus according to any one of claim 7 to claim 9, wherein
the dividing unit presses the one surface of the small piece using the support member in the crossing direction.

11. The tissue dividing apparatus according to claim 10, wherein
the support member supports the surface to which the section is pasted via the section,
and
a protective member is provided between the support member and the section so as to cover and protect the surface of the section.

12. The tissue dividing apparatus according to any one of claim 7 to claim 9, further comprising:
an attracting member that is disposed between the support member and the opposite surface of the part of the small pieces and that attracts the opposite surface,
wherein the dividing unit pulls the opposite surface of the part of the small pieces in the crossing direction using the support member.

13. The tissue dividing apparatus according to any one of claim 7 to claim 9, wherein
the dividing unit comprises two support members that are oppositely disposed to each other with the substrate held therebetween and thereby support both the surfaces of the small pieces to which the section is pasted and the opposite surface, and that integrally move in the crossing direction.

14. A cell collection apparatus comprising:
a substrate having a surface to which a section of a biological tissue can be pasted, and dividable into a plurality of small pieces;
a dividing unit that divides the substrate into the plurality of small pieces, and thereby divides the section pasted on the surface into fragments each having a substantially equivalent shape to a shape of each small piece; and
a pickup unit that picks up the fragments of the section divided by the dividing unit.

15. A cell collecting system comprising:
a cell collection apparatus according to claim 14; and
an observation device that observes the section on the substrate.

16. A tissue displaying system comprising:
the cell collection apparatus according to claim 14;
an observation apparatus that observes the section on the substrate; and
a displaying unit that displays the section observed by the observation apparatus.

17. A substrate configured to: have a surface to which a section of a biological tissue can be pasted; be dividable into a plurality of small pieces; and divide the section pasted on the surface by being divided into the plural small pieces.

18. An expandable member configured to:
be adhered to an opposite surface to a surface to which a section of a biological tissue can be pasted of a substrate dividable into a plurality of small pieces;
be expandable in a surface direction of the substrate; and
expand in the surface direction so as to divide the substrate where the expandable member is adhered into the plurality of small pieces.

19. A tissue dividing method comprising:
a pasting step of pasting a section of a biological tissue on a substrate which has a surface to which the section of the biological tissue can be pasted, and is dividable into a plurality of small pieces in such a manner that the section is pasted on the surface of the substrate across the plurality of small pieces; and
a dividing step of dividing the section pasted on the surface of the substrate by dividing the substrate into the plurality of small pieces.

20. A cell collecting method comprising:
a pasting step of pasting a section of a biological tissue on a substrate which has a surface to which the section can be pasted, and is dividable into a plurality of small pieces;
a dividing step of dividing the substrate to which the section is pasted into the plurality of small pieces, to thereby divide the section into fragments each having a substantially equivalent shape to a shape of each small piece; and
a pickup step of picking up the fragments of the divided section.
